# EUROPEAN PATENT SPECIFICATION

(11) **EP 1 896 405 B1**
(45) Date of publication and mention of the grant of the patent: **10.08.2011**
(21) Application number: 06752578.2
(22) Date of filing: 14.06.2006
(51) Int. Cl.: C07C 323/52, C07D 211/60, C07D 211/54, A61K 31/165, A61K 31/16, A61P 17/10

(54) **ORGANIC COMPOUNDS**
ORGANISCHE VERBINDUNGEN
COMPOSES ORGANIQUES

(30) Priority: 27.06.2005 GB 0513058
(43) Date of publication of application: 12.03.2008
(73) Proprietor: Nabriva Therapeutics AG, 1112 Wien (AT)
(72) Inventor: ASCHER, Gerd, A-6250 Kundl (AT)
(74) Representative: Schwarz, Albin
(86) International application number: PCT/AT2006/000242
(87) International publication number: WO 2007/000001

(56) References cited:
- WO-A-01/09095
- WO-A-02/04414
- WO-A-02/22580
- WO-A-99/21855
- WO-A-03/082260
- WO-A-2004/089886
- WO-A-2005/023257

## Description

The present invention relates to mutilins, such as pleuromutilins, which are useful as antimicrobials, e.g. antibiotics.

We have now found salts of pleuromutilins which exhibit surprising activity.

In one aspect the present invention provides a compound of formula 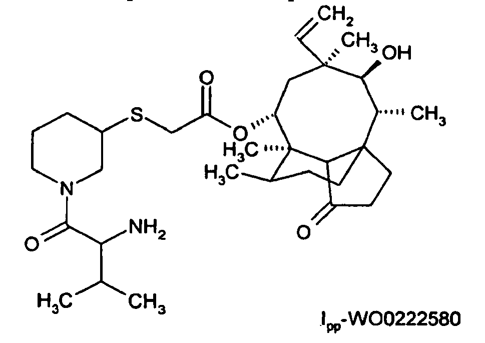 in the form of a salt with salicylic acid, azelaic acid, sebacic acid or 2-[(2,6-dichlorophenyl)amino]benzeneacetic acid, preferably salicylic acid, such as the compound14-O-[(N-((R)-valyl)-piperidine-3(S)-yl)-sulfanylacetyl)-mutilin in the form of a salt with salicylic acid, azelaic acid, sebacic acid or 2-[(2,6-dichlorophenyl)amino]benzeneacetic acid, preferably salicylic acid. 2-[(2,6-dichlorophenyl)amino]benzeneacetic acid is also known as diclofenac.

A compound of formula Iₚₚ-WO0222580 such as the compound14-O-[(N-((R)-valyl)-piperidine-3(S)-yl)-sulfanylacetyl)-mutilin, in the form of a salt with salicylic acid, azelaic acid, sebacic acid or 2-[(2,6-dichlorophenyl)amino]benzeneacetic acid are herein also designated as "compound(s) of (according to) the present invention".

A pleuromutilin in a compound of the present invention may exist in the form of isomers and mixtures thereof, e.g. including diastereoisomers and mixtures thereof. Isomeric mixtures may be separated as appropriate, e.g. according to a method as conventional, to obtain pure isomers. The present invention includes a pleuromutilin in a compound of the present invention in any isomeric form and in any isomeric mixture, such as described in patent literature cited below. Preferably the configuration in the mutilin ring is the same as in a naturally produced mutilin.

Pleuromutilin, e.g. a compound of formula 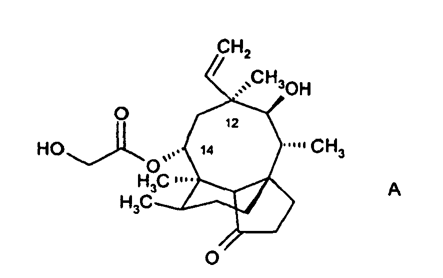 is a naturally occurring antibiotic, e.g. produced by the basidomycetes Pleurotus mutilus and P. passeckerianus, see e.g. The Merck Index, 12th edition, item 7694. A number of further pleuromutilins having the principle ring structure of pleuromutilin and having e.g. antibacterial activity are known.

The pleuromutin in a compound of the present invention is e.g. disclosed in W00222580, e.g. including compounds of formula 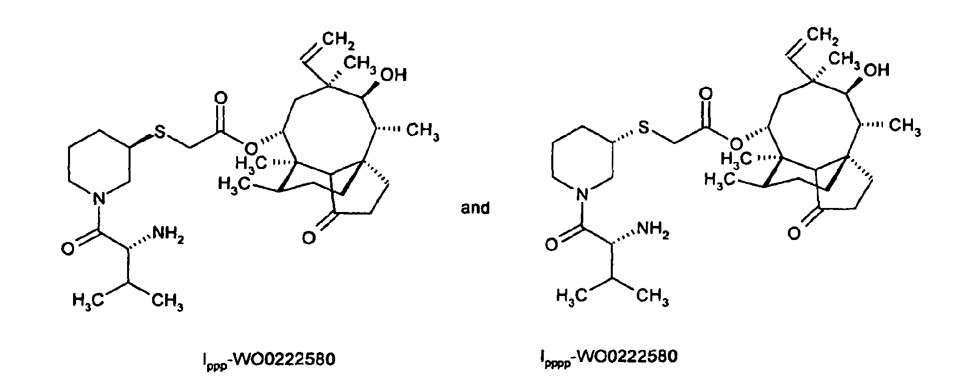 e.g. wherein the group attached to the pleuromutilin via the sulphur atom is either in the S-configuration or in the R-configuration.

A compound of the present invention may be prepared as appropriate, e.g. according, e.g analogously, to a method as conventional; e.g. a pleuromutilin in a compound of the present invention may be dissolved or suspended in appropriate solvent, preferably organic solvent, e.g. including halogenated hydrocarbons, such as CH₂Cl₂, an alcohol, such as methanol, ethanol, an ether, such as dimethylether, diisopropylether or tetrahydrofuran, a ketone, such as acetone, and the suspension or solution obtained may be treated with salicylic acid, azelaic acid, sebacic acid or 2-[(2,6-dichlorophenyl)amino]benzeneacetic acid, e.g. as such, e.g. or dissolved or suspended in a solvent, e.g. in an amount that is at least equivalent to the amount of the pleuromutilin used. From the mixture obtained a compound of the present invention may be isolated as appropriate, e.g.
- from the mixture obtained a solid may precipitate, e.g. in crystalline form, which may be isolated by filtration, centrifugation,
- from the mixture obtained solvent may be evaporated off and the evaporation residue may be collected,
- the mixture obtained may be subjected to lyophilisation.

A compound of the present invention obtained may be purified as appropriate, e.g. according, e.g. analogously, to a method as conventional, e.g. by extraction and/or by chromatography; e.g. before or after isolation

The compounds of the present invention exhibit pharmacological activity and are therefore useful as pharmaceuticals, such as antimcrobials or antibiotics, in the treatment of tuberculosis, in the treatment of Helicobacter infections. Such activity is e.g. described in correspondingly cited literature.

We have found that compounds of the present invention are particularly useful in the treatment of acne. E.g. in commercially available in vitro acne assays (the determination of the minimal inhibitory concentration (MIC) is performed according to the approved NCCLS guidelines¹⁾ by agar dilution technique on Wilkins-Chalgren Agar - 1) National committee for clinical laboratory standards. Methods for Antimicrobial susceptibility testing of anaerobic bacteria. 1993. (NCCLS document M11-A3 3rd edition. Vol. 13, No. 26) the compound 14-O-[(N-((R)-Valyl)-piperidine-3(S)-yl)-sulfanylacetyl)-mutilin in the form of a salt with salicylic acid shows remarkably better activity than the compound 14-O-[(N-((R)-Valyl)-piperidine-3(S)-yl)-sulfanylacetyl)-mutilin in the form of a hydrochloride.

In another aspect the present invention provides a compound of formula Iₚₚ-WO0222580, such as a compound of formula Iₚₚₚ-WO0222580 or Iₚₚₚₚ-WO0222580, e.g. wherein the group attached to the pleuromutilin via the sulphur atom is either in the S-configuration or in the R-configuration, in the form of a salt with salicylic acid, azelaic acid, sebacic acid or 2-[(2,6-dichlorophenyl)amino]benzeneacetic acid, preferably salicylic acid, such as the compound 14-O-[(N-((R)-Valyl)-piperidine-3(S)-yl)-sulfanylacetyl)-mutilin in the form of a salt with salicylic acid, azelaic acid, sebacic acid or 2-[(2,6-dichlorophenyl)amino]benzeneacetic acid, preferably salicylic acid, for use in the treatment of acne, e.g..for use in the preparation of a medicament for the treatment of acne.

A compound of the present invention is useful in a method of treating acne, comprising administering an effective amount of a compound of
formula Iₚₚₚ-WO0222580 or Iₚₚₚₚ-WO0222580, e.g. wherein the group attached to the pleuromutilin via the sulphur atom is either in the S-configuration or in the R-configuration, in the form of a salt with salicylic acid, azelaic acid, sebacic acid or 2-[(2,6-dichlorophenyl)amino]benzeneacetic acid, preferably salicylic acid, such as the compound 14-O-[(N-((R)-Valyl)-piperidine-3(S)-yl)-sulfanylacetyl)-mutilin in the form of a salt with salicylic acid, azelaic acid, sebacic acid or 2-[(2,6-dichlorophenyl)amino]benzeneacetic acid, preferably salicylic acid, to a subject in need of such treatment.

In another aspect the present invention provides a pharmaceutical composition for the treatment of acne comprising a compound of formula Iₚₚ-WO0222580, such as a compound of formula Iₚₚₚ-WO0222580 or Iₚₚₚₚ-WO0222580, e.g. wherein the group attached to the pleuromutilin via the sulphur atom is either in the S-configuration or in the R-configuration, in the form of a salt with salicylic acid, azelaic acid, sebacic acid or 2-[(2,6-dichlorophenyl)amino]benzeneacetic acid, preferably salicylic acid, such as the compound 14-O-[(N-((R)-Valyl)-piperidine-3(S)-yl)-sulfanylacetyl)-mutilin in the form of a salt with salicylic acid, azelaic acid, sebacic acid or 2-[(2,6-dichlorophenyl)amino]benzeneacetic acid, preferably salicylic acid, in association with at least one pharmaceutical excipient, e.g. appropriate carrier and/or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

For pharmaceutical use a compound of the present invention includes one or more, preferably one, compounds of the present invention, e.g. a combination of two or more compounds of the present invention.

Treatment includes treatment and prophylaxis.

For such treatment, the appropriate dosage will, of course, vary depending upon, for example, the chemical nature and the pharmakokinetic data of a compound of the present invention employed, the individual host, the mode of administration and the nature and severity of the conditions being treated. However, in general, for satisfactory results in larger mammals, for example humans, an indicated daily dosage is in the range from about 0.01 g to about 1.0 g of a compound of the present invention; conveniently administered, for example, in divided doses up to four times a day.

A compound of the present invention may be administered by any conventional route, for example enterally, e.g. including nasal, buccal, rectal, oral, administration; parenterally, e.g. including intravenous, intramuscular, subcutaneous administration; or topically; e.g. including epicutaneous, intranasal, intratracheal administration;
e.g. in form of coated or uncoated tablets, capsules, (injectable) solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.

A compound of the present invention may be used for pharmaceutical treatment according to the present invention alone, or in combination with one or more other pharmaceutically active agents. Such other pharmaceutically active agents include agents which are appropriate for the treatment of inflammation, e.g. including skin inflammation, including agents useful for the treatment of acne.

In another aspect the present invention provides a pharmaceutical composition according to the present invention further comprising another agent which is appropriate in the treatment of skin inflammation, e.g. acne.

Combinations include fixed combinations, in which two or more pharmaceutically active agents are in the same formulation; kits, in which two or more pharmaceutically active agents in separate formulations are sold in the same package, e.g. with instructions for coadministration; and free combinations in which the pharmaceutically active agents are packaged separately, but instructions for simultaneous or sequential administration are given.

Such compositions may be manufactured according, e.g. analogously to a method as conventional, e.g. by mixing, granulating, coating, dissolving or lyophilizing processes. Unit dosage forms may contain, for example, from about 0.5 mg to about 1000 mg, such as 1 mg to about 500 mg.

In the following Examples all temperatures are in degrees Celsius (°C) and are uncorrected.

### Example 1

### 14-O-[(N-((R)-Valyl)-piperidine-3(S)-yl)-sulfanylacetyl)-mutilin in the form of a salt with salicylic acid

1.38g (10 mmol) of salicylic acid are added to a stirred cooled (5°) solution of 5.76 g (10 mmol) of 14-O-[(N-((R)-Valyl)-piperidine-3(S)-yl)-sulfanyl-acetyl)-mutilin in 30 ml of CH₂Cl₂. The mixture obtained is stirred for 15 minutes at room temperature and from the mixture obtained solvent is evaporated to dryness under reduced pressure. Amorphous material obtained is dried for further 5 hours at reduced pressure.
14-O-[(N-((R)-Valyl)-piperidine-3(S)-yl)-sulfanylacetyl)-mutilin in the form of a salt with salicylic acid is obtained.
¹HNMR (DMSO-d₆, ~1:1 mixture of stable rotamers):
7.7-8.3 (b, 3H, NH3⁺), 6.6, 7.1, 7.6 (3 x m, 4H, salicylate), 6.15 (2xdd,
1H, H19, J=17.6Hz, J=11.2Hz), 5.6 (2xd, 1H, H14, J=8.2Hz), 5.05 (m, 2H, H20),
4.5 (b, 1H, 11-OH), 4.08 (dd, 0.5H, H2-Piperidin, J=13.7Hz, J=3.3Hz),
3.08 (dd, 0.5H, H2-Piperidin, J=13.7Hz, J=9.8Hz), 3.89 (dd, 0.5H, H2- Piperidin,
J=13.1Hz, J=3.2Hz), 3.41 (m, 0.5H, H2-Piperidin), AB-System: *v*_{A}=3.44, *v*_{B}=3.33 (2H, SCH₂,J=14.9Hz), 2.83,2.96 (2xm, 1H, CHS), 2.4 (b, 1H, H4), 1.34 (s, 3H, CH₃-15),
1.05 (s, 1H, CH₃-18), 0.9, 1.0 (2xm, 6H, (CH₃)₂-Val), 0.81 (d, 3H, CH₃-17, J=6.9Hz), 0.63 (m, 3H, CH₃-16).

### Example 2

### 14-O-[(N-((R)-Valyl)-piperidine-3(S)-yl)-sulfanylacetyl)mutilin in the form of a salt

### with sebacic acid

The salt is prepared analogously to Example 1.
¹H-NMR (DMSO-d₆, ~ 1:1 mixture of stable rotamers):
6.12 (2xdd, 1H, H-19, J=17.1 Hz, J=11.1 Hz), 5.55 (2xd, 1H, H-14, J=8.6 Hz), 5.05 (m, 2H, H-20), 4.5 (b, 1H, 11-OH), 3.98 (m, 1.5H, H-2 piperidine), 3.49 (m,1.SH, H-2 piperidine, α-H Val), 3.42 (d, 1H, H-11, J=6Hz), 3.30 (m, 0.5H, H-2 piperidine), 3.27(m, 2H, H-22), 3.10 (2xm, 2x0.5H, H-2 piperidine), 2.86 (m, 1.5H, H-2 piperidine, CH-S), 2.4, (b,1H, H-4), 2.22 - 2.02 (m, 4H, H-2, 10, 13a), 2,14 (t, 4H, α-H sebacate), 1.92-1.98 (m,1H, H-4 piperidine), 1.58-1.72 (m, 4H, β-H Val, 1a, 8a, H-3 piperidine), 1.42-1.52 (m,8H, H-6, 7a, H-3 piperidine, H-4 piperidine, β -H sebacate), 1.34 (s, 3H, CH₃-15), 1.20-1.32 (m, 11H, H-1, 7, 13, γ-H sebacate, δ-H sebacate), 1.05 (s, 3H, CH₃-18), 1.00 (m, 1H, H-8), 0.87, 0.75 (2xm, 6H, (CH₃)₂-Val), 0.80 (d, 3H, CH₃-17, J = 6.8 Hz), 0.62 (m, 3H, CH₃-16).

### Example 3

### 14-O-[(N-((R)-Valyl)-piperidine-3(S)-yl)-sulfanylacetyl)mutilin in the form of a salt with azelaic acid

The salt is prepared analogously to Example 1.
¹H-NMR (DMSO-d₆, ~1:1 mixture of stable rotamers):
6.12 (2xdd, 1H, H-19, J=17.1 Hz, J=11.1 Hz), 5.55 (2xd, 1H, H-14, J=8.6 Hz), 5.05 (m, 2H, H-20), 4.5 (b, 1H, 11-OH), 3.98 (m, 1.5H, H-2 piperidine), 3.49 (m,1.5H, H-2 piperidine, α-H Val), 3.42 (d, 1H, H-11, J=6Hz), 3.30 (m, 0.5H, H-2 piperidine), 3.27(in, 2H, H-22), 3.10 (2xm, 2x0.5H, H-2 piperidine), 2.86 (m, 1.5H, H-2 piperidine, CH-S), 2.4, (b,1H, H-4), 2.22 - 2.02 (m, 4H, H-2, 10, 13a), 2,14 (t, 4H, α-H azelate), 1.92-1.98 (m, 1H, H-4 piperidine), 1.58-1.72 (m, 4H, β-H Val, 1a, 8a, H-3 piperidine), 1.42-1.52 (m, 8H, H-6, 7a, H-3 piperidine, H-4 piperidine, β -H azelate), 1.34 (s, 3H, CH₃-15), 1.20-1.32 (m, 9H, H-1, 7, 13, γ-H azelate, δ-H azelate), 1.05 (s, 3H, CH₃-18), 1.00 (m, 1H, H-8), 0.87, 0.75 (2xm, 6H, (CH₃)₂-Val), 0.80 (d, 3H, CH₃-17, J = 6.8 Hz), 0.62 (m, 3H, CH₃-16).

## Claims

1. A compound of formula 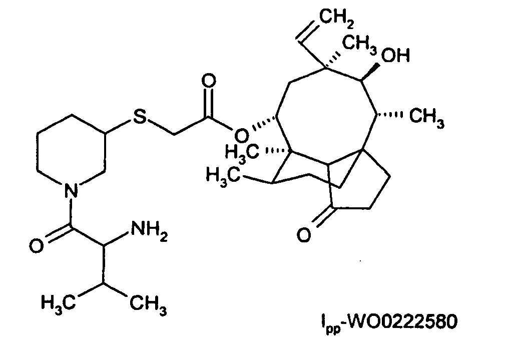 in the form of a salt with salicylic acid, azelaic acid, sebacic acid or 2-[(2,6-dichlorophenyl)amino]benzeneacetic acid.

2. The compound according to claim 1 which is 14-O-[(N-((R)-valyl)-piperidine-3(S)-yl)-sulfanylacetyl)-mutilin in the form of a salt with salicylic acid, azelaic acid, sebacic acid or 2-[(2,6-dichlorophenyl)amino]benzeneacetic acid.

3. A compound according to any preceding claim in the form of a salt with salicylic acid.

4. A compound as defined in any one of claims 1 to 3 for use in the preparation of a medicament for the treatment of acne.

5. A compound as defined in any one of claims 1 to 3 for use in the treatment of acne.

6. A pharmaceutical composition for the treatment of acne, comprising a compound as defined in any one of claims 1 to 3 in association with at least one pharmaceutical excipient

## Patentansprüche

1. Verbindung der Formel 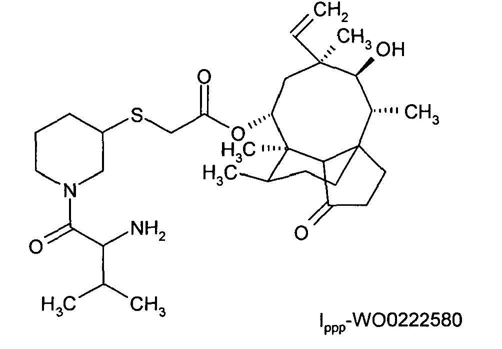 in der Form eines Salzes mit Salicylsäure, Azelainsäure, Sebacinsäure oder 2-[(2,6-Dichlorphenyl)amino]benzolessigsäure.

2. Verbindung nach Anspruch 1, welche 14-O-[(N-((R)-Valyl)-piperidin-3(S)-yl)-sulfanylacetyl]-mutilin in der Form eines Salzes mit Salicylsäure, Azelainsäure, Sebacinsäure oder 2-[(2,6-Dichlorphenyl)amino]benzolessigsäure ist.

3. Verbindung nach einem der vorstehenden Ansprüche in der Form eines Salzes mit Salicylsäure.

4. Verbindung, wie sie nach einem der Ansprüche 1 bis 3 definiert ist, zur Verwendung in der Herstellung eines Medikaments zur Behandlung von Akne.

5. Verbindung, wie sie in einem der Ansprüche 1 bis 3 definiert ist, zur Verwendung in der Behandlung von Akne.

6. Pharmazeutische Zusammensetzung zur Behandlung von Akne, umfassend eine Verbindung, wie sie in einem der Ansprüche 1 bis 3 definiert ist, in Verbindung mit zumindest einem pharmazeutischen Arzneistoffträger.

## Revendications

1. Composé de formule 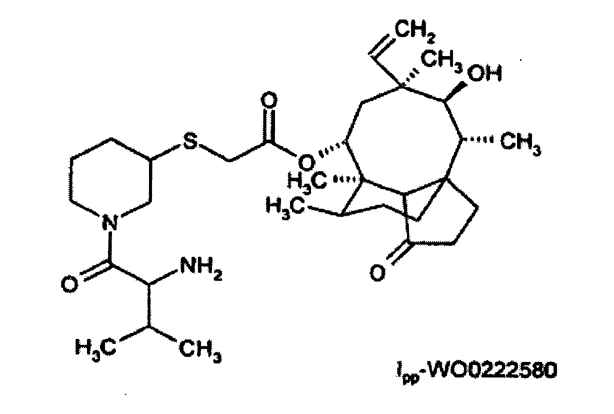 sous la forme d'un sel avec l'acide salicylique, l'acide azélaïque, l'acide sébacique ou l'acide 2-[(2,6-dichlorophényl)amino]benzèneacétique.

2. Composé selon la revendication 1 qui est la 14-0-[(N-((R)-valyl)-pipéridine-3(S)-yl)-sulfanylacétyl)-mutiline sous la forme d'un sel avec l'acide salicylique, l'acide azélaïque, l'acide sébacique ou l'acide 2-[(2,6-dichlorophényl)amino]benzèneacétique.

3. Composé selon une quelconque revendication précédente sous la forme d'un sel avec l'acide salicylique.

4. Composé selon l'une quelconque des revendications 1 à 3 pour son utilisation dans la préparation d'un médicament pour le traitement de l'acné.

5. Composé selon l'une quelconque des revendications 1 à 3 pour son utilisation dans le traitement de l'acné.

6. Composition pharmaceutique pour le traitement de l'acné, comprenant un composé selon l'une quelconque des revendications 1 à 3 en association avec au moins un excipient pharmaceutique.
